# FASCICULE DE BREVET EUROPEEN

(11) **EP 2 316 021 B1**
(45) Date de publication et mention de la délivrance du brevet: **19.09.2012**
(21) Numéro de dépôt: 09781584.9
(22) Date de dépôt: 06.08.2009
(51) Int. Cl.: G01N 33/08

(54) **Procédé de mirage d'oeufs et dispositif correspondant**
Eierdurchleuchtungsverfahren und entsprechende Vorrichtung
Egg candling method and corresponding device

(30) Priorité: 13.08.2008 FR 0855571
(43) Date de publication de la demande: 04.05.2011
(73) Titulaire: Egg-Chick Automated Technologies, 35740 Pace (FR)
(72) Inventeur: ADJANOHOUN, Ephrem, F-35740 Pace (FR)
(74) Mandataire: Novagraaf Technologies
(86) Numéro de dépôt international: PCT/EP2009/060242
(87) Numéro de publication internationale: WO 2010/018127

(56) Documents cités:
- FR-A- 786 607
- FR-A- 2 787 579
- FR-A- 2 912 217
- JP-A- 2001 027 612
- JP-A- 2004 101 204
- US-A- 5 173 737
- US-A- 5 745 228
- US-A1- 2006 038 978

## Description

La présente invention concerne un procédé de mirage d'oeufs pour déterminer un état desdits oeufs. L'invention concerne également un dispositif apte à mettre en oeuvre ce procédé.

Dans l'industrie de la volaille, notamment la production de poussins, il est connu des procédés de mirage automatique d'oeufs (cf. les demandes de brevet japonaises JP 2004 101 204 A, française FR 2 912 217 A et américaine US 2006/038978 A1) utilisant la transparence de l'oeuf à la fin de la période d'incubation pour différencier deux catégories d'oeufs: les oeufs pleins opaques à la lumière comprenant non seulement les oeufs fécondés contenant un embryon complètement développé vivant ou mort, mais également les oeufs contaminés ou pourris, et les oeufs perméables à la lumière comprenant les oeufs non fécondés, dits oeufs clairs, mais aussi les oeufs peu développés contenant un embryon mort précocement. Ces procédés consistent à émettre un flux lumineux en direction d'un oeuf à mirer et à analyser ensuite le flux lumineux passé à travers l'oeuf pour en déterminer son état, fécondé ou non fécondé, en fonction du taux de flux lumineux absorbé par l'oeuf. Pour ce faire, le système comporte de manière générale des moyens d'émission d'un flux lumineux, des moyens de réception du flux lumineux passé à travers l'oeuf et des moyens de traitement informatique du flux lumineux reçu par les moyens de réception. Les moyens de traitement informatique sont alors capables de différencier grossièrement les oeufs en fonction du taux du flux lumineux absorbé par l'oeuf. A la fin de la période d'incubation, l'oeuf fécondé renfermant un embryon vivant normalement développé est opaque. Mais, c'est aussi le cas des oeufs renfermant un embryon mort tardivement ou des oeufs contaminés et pourris. Ces derniers ne sont donc pas facilement différentiables des oeufs normalement développés. Quant aux oeufs qui sont perméables à la lumière à la fin de la période d'incubation, ils correspondent soit aux oeufs infertiles, soit aux oeufs renfermant un embryon mort précocement, sans qu'il soit possible de les distinguer de manière fiable.

Ce procédé de mirage par transparence ne permet donc pas de différencier avec certitude les oeufs vivants des oeufs morts à la fin de la période d'incubation. Il n'est pas non plus adapté pour être utilisé à un stade plus précoce du cycle de développement de l'oeuf, par exemple entre le 3^{ème} et le 17^{ème} jour du cycle d'incubation de l'oeuf de poule. Il ne permet pas de connaître avec certitude l'état fécondé ou non fécondé de l'oeuf en début de cycle du fait de la taille insignifiante de l'embryon. Il ne permet pas non plus de fournir des informations plus précises sur l'état vivant ou mort de l'embryon à un stade intermédiaire du cycle d'incubation et/ou sur son état de développement (développement normal ou pas de l'embryon à ce même stade intermédiaire du cycle d'incubation). Par ailleurs, il n'est pas non plus adapté pour déterminer un état retourné ou non retourné de l'oeuf en fonction de la position de sa chambre à air. Enfin, il ne donne aucune information sur l'intégrité de la coquille de l'oeuf, à savoir la présence ou non de fêlures sur la coquille.

De façon alternative, un mirage effectué manuellement à l'aide d'une source de lumière blanche ne permet pas non plus de pallier les inconvénients précités en raison de la visibilité médiocre des structures internes de l'oeuf.

Le but de la présente invention est de proposer une solution palliant tout ou partie des inconvénients précités.

A cet effet, la présente invention a pour objet un procédé de mirage d'oeufs pour déterminer au moins un état d'au moins un oeuf fécondé contenant un embryon, comportant les étapes suivantes:
a) éclairement d'au moins une partie dudit oeuf avec une source de lumière bleue ayant une ou plusieurs longueurs d'onde comprises entre 440 et 510 nm, de manière à faire apparaître visuellement sur la coquille de l'oeuf une ombre portée d'un éventuel réseau sanguin de l'embryon et capture d'au moins une image dudit oeuf éclairé; et
b) analyse de ladite image pour déterminer en fonction de la présence ou non d'un réseau sanguin alimentant l'embryon dudit oeuf dans ladite image et, en cas de présence d'un réseau sanguin, de la dimension des veines dudit réseau sanguin, un niveau de vitalité de l'embryon dudit oeuf.

On peut notamment en déduire si l'embryon est vivant ou mort ou si son niveau de vitalité est normal ou pas en fonction de son âge.

Cet intervalle de longueurs d'ondes correspond à une lumière bleue. L'utilisation de cette lumière bleue avantageusement sous forme diffractée, pennet de faire apparaître très distinctement sur la coquille de l'oeuf l'ombre des veines et des vaisseaux du réseau sanguin alimentant l'embryon de l'oeuf du fait qu'ils sont positionnés contre les parois et membranes internes de la coquille.

Dans une variante ne faisant pas partie de la présente invention, on utilise une lumière verte dont longueur d'onde est comprise entre 510 et 570 nm, de préférence entre 530 nm et 550 nm. Cette longueur d'onde peut être utilisée entre le 3^{ème} et le 17^{ème} jour du cycle d'incubation

Dans la présente invention, on utilise, avantageusement à un stade précoce du cycle H d'incubation, par exemple entre le 3^{ème} et le 6^{ème} jour d'incubation, une lumière bleue dont la longueur d'onde est comprise entre 440 et 510 nm.

Typiquement, cette lumière bleue fait apparaître principalement 3 zones de haut en bas de l'oeuf: une première zone très lumineuse correspondant à la chambre à air, une deuxième zone de luminosité intermédiaire qui correspond à la zone de l'oeuf contenant essentiellement les fluides embryonnaires, le réseau sanguin alimentant l'embryon vivant ou mort et, en position basse de cette zone, une masse opaque correspondant à une partie du corps de l'embryon et enfin une troisième zone très sombre dans laquelle aucune structure n'est identifiable.

Pendant l'étape d'analyse de ladite image, on détecte dans l'image la présence ou non d'un réseau de lignes sombres entrecroisées représentant l'ombre portée du réseau sanguin et on en déduit, en fonction de la présence ou non de ces lignes sombres et de leur dimension (épaisseur et/ou longueur), le niveau de vitalité de l'embryon.

Selon une particularité de l'invention, l'étape a) comprend l'éclairement de la partie supérieure de l'oeuf, disposé de sorte que son axe de révolution soit sensiblement vertical, de préférence au moyen d'une source de lumière disposée au-dessus de l'oeuf sensiblement sur l'axe de révolution de l'oeuf, et la capture d'une image de la partie supérieure de l'oeuf éclairé par une caméra, disposée à proximité de la source de lumière, l'axe de prise de vue de la caméra formant un angle compris entre 0 et 70° avec un plan de référence perpendiculaire à l'axe de révolution de l'oeuf et situé à mi-distance entre l'extrémité aplatie et l'extrémité pointue de l'oeuf.

Avantageusement, on prévoit la capture d'au moins deux images de l'oeuf éclairé, par au moins deux caméras distinctes couvrant avantageusement les deux cotés de l'oeuf pour s'assurer que le réseau sanguin, s'il est présent, apparaisse visuellement sur au moins l'une des images capturées quelle que soit sa position dans l'oeuf. Dans ce cas, l'étape a) comprend l'éclairement de l'oeuf avec une lumière bleue, la capture par une première caméra d'une image I1 de l'oeuf éclairé et la capture par une deuxième caméra d'une image I2 de l'oeuf éclairé, les première et deuxième caméras étant disposées en vis-à-vis de part et d'autre d'un plan médian de l'oeuf comprenant l'axe de révolution de l'oeuf. L'étape b) comprend alors une analyse des deux images I1 et I2 pour déterminer le niveau de vitalité de l'embryon de l'oeuf.

Selon un deuxième mode de réalisation, on prévoit d'exciter l'embryon contenu dans l'oeuf et de capturer une image de l'oeuf éclairé avant et après excitation pour déterminer si le réseau sanguin de l'oeuf a changé de position et en déduire si l'oeuf est vivant ou mort. En effet, si l'oeuf est vivant, il réagit au stimulus et la position de son réseau sanguin ainsi que celle de la masse opaque du corps de l'embryon s'en trouve modifiée. Dans ce mode de réalisation, l'étape a) comprend l'éclairement de l'oeuf avec une lumière bleue, la capture d'une image I1 de l'oeuf éclairé par une caméra, l'excitation de l'embryon de l'oeuf par un stimulus et, après excitation, l'éclairement de l'oeuf avec ladite lumière bleue et la capture d'une image I'1 de l'oeuf éclairé par la même caméra. L'étape b) du procédé de l'invention comprend une comparaison des deux images I1 et I'1 pour déterminer l'état vivant ou l'état mort de l'oeuf et/ou confirmer le niveau de vitalité de l'embryon. Si les images I1 et I'1 sont différentes, l'oeuf est dans un état vivant.

Ce mode de réalisation avec excitation de l'embryon est utilisé avec l'analyse préalable de la dimension des veines du réseau pour déterminer un niveau de vitalité de l'embryon dudit oeuf.

Avantageusement, dans ce mode de réalisation, on capture au moins deux images par deux caméras distinctes avant l'excitation de l'embryon et au moins deux images par ces mêmes caméras après excitation pour s'assurer que le réseau sanguin, s'il est présent dans l'oeuf, soit visible sur au moins une des images avant excitation et après excitation. Dans ce cas, l'étape a) comprend l'éclairement de l'oeuf avec une lumière bleue, la capture d'une image I1 de l'oeuf éclairé par une première caméra et la capture d'une image I2 de l'oeuf éclairé par une deuxième caméra, l'excitation de l'embryon de l'oeuf par un stimulus, et, après excitation, l'éclairement de l'oeuf avec ladite lumière bleue, la capture d'une image I'1 de l'oeuf éclairé par la première caméra et la capture d'une image I'2 de l'oeuf éclairé par la deuxième caméra. L'étape b) du procédé de l'invention comprend une comparaison des deux images I1 et I'1 et/ou une comparaison des deux images I2 et I'2 pour déterminer l'état vivant ou l'état mort de l'oeuf et/ou confirmer le niveau de vitalité de l'embryon.

Pour l'étape d'excitation, le stimulus employé est une perturbation temporaire du milieu environnant l'embryon déclenchant ou amplifiant son mouvement dans l'oeuf. Il peut prendre par exemple la forme d'une onde lumineuse, sonore et/ou thermique ou d'une vibration provoquée par exemple par un choc ou tout autre type de stimulus provoquant un mouvement chez l'embryon. Le stimulus est par exemple un signal lumineux intense, et/ou thermique, tel qu'une lumière blanche ou un signal laser.

Selon un perfectionnement applicable à tous les modes de réalisation, l'étape b) comprend en outre une analyse de l'image ou des images pour déterminer la présence ou non de la chambre à air en partie supérieure de l'oeuf en déduire si l'oeuf est dans un état retourné ou un état non retourné et éventuellement si la chambre à air est décalée par rapport a l'axe vertical de l'oeuf.

Selon un perfectionnement applicable à tous les modes de réalisation, l'étape b) comprend en outre une analyse de l'image ou des images pour analyser l'intégrité de la coquille et repérer des fêlures laissant s'échapper la lumière de façon plus intense.

Selon un mode de réalisation particulier, pour deux oeufs voisins, l'éclairement est effectué de manière alternée entre les deux oeufs.

L'invention concerne également un dispositif de mirage automatique d'oeufs pour déterminer au moins un état d'un oeuf fécondé, comprenant :
- des moyens d'éclairement comprenant au moins une source de lumière bleue ayant une ou plusieurs longueurs d'onde comprises entre 440 nm et 510 nm, pour éclairer ledit oeuf de manière à faire apparaître visuellement sur la coquille dudit oeuf une ombre portée du réseau sanguin de l'embryon de l'oeuf;
- des moyens de capture d'image pour capturer au moins une image de l'oeuf éclairé; et
- des moyens d'analyse d'image pour déterminer, en fonction de la pensée ou non d'un réseau sanguin alimentant l'embryon de l'oeuf dans la dite image et, en cas de présence d'un réseau sanguin, de la dimension des veines dudit réseau sanguin, un niveau de vitalité de l'embryon dudit oeuf.

Selon un mode de réalisation particulier, le dispositif comprend en outre :
- des moyens de convoyage pour transporter, selon un plan de convoyage et une direction d'avancement, des plateaux comportant des alvéoles dans lesquelles sont disposés des oeufs à mirer,
- un support, disposé au-dessus des moyens de convoyage, portant de manière fixe les moyens d'éclairement comprenant au moins une rampe de sources de lumière et les moyens de capture d'image comprenant au moins une rampe de caméras, ladite rampe de sources de lumière et ladite rampe de caméras étant disposées parallèlement ou transversalement à la direction d'avancement, et
- des moyens de déplacement aptes à déplacer verticalement ledit support entre une position relevée et une position de mirage dans laquelle les sources de lumière viennent en appui sur la partie supérieure des oeufs positionnés en vis-à-vis dans les alvéoles.

Avantageusement, les moyens de déplacement sont aptes à déplacer le support parallèlement et/ou transversalement à la direction d'avancement et/ou les moyens de capture d'image comportent deux rampes de caméras disposées symétriquement de part et d'autre de la rampe des sources de lumière, l'axe de prise de vue des caméras formant un angle compris entre 0 et 70° avec le plan de convoyage des plateaux. Ce dispositif permet de maintenir la même distance entre les cameras et l'oeuf, quelle que soit la position de l'oeuf dans les plateaux et quel que soit le type des plateaux.

Avantageusement, les moyens d'éclairement comprennent des moyens pour faire varier la luminosité (intensité lumineuse) de la source de lumière.

Avantageusement, les moyens d'éclairement comprennent également des moyens pour guider la lumière produite par la source de lumière vers l'oeuf de façon hermétique de façon à ne pas polluer le milieu environnant l'oeuf.

L'invention sera mieux comprise, et d'autres buts, détails, caractéristiques et avantages apparaîtront plus clairement au cours de la description explicative détaillée qui va suivre de deux modes de réalisation particuliers actuellement préférés de l'invention, en référence aux dessins schématiques annexés, sur lesquels :
- la figure 1 représente un schéma de principe illustrant le procédé de l'invention;
- la figure 2 représente un organigramme montrant les étapes du procédé de l'invention selon un premier mode de réalisation de l'invention;
- la figure 3 représente un organigramme montrant les étapes du procédé de l'invention selon un deuxième mode de réalisation de l'invention;
- la figure 4 représente une vue de côté, perpendiculaire à la direction d'avancement des plateaux, d'un dispositif apte à mettre en oeuvre le procédé de l'invention, dans lequel le support de la rampe des sources de lumière et de la rampe de caméras est en position relevée;
- la figure 5 représente une vue de côté, parallèle à la direction d'avancement des plateaux, du dispositif représenté à la figure 4, et
- la figure 6 représente une vue analogue à celle de la figure 4, dans lequel le support de la rampe de sources de lumière et de la rampe de caméras est en position de mirage.

Selon l'invention, l'oeuf est éclairé avec une lumière bleue pour faire apparaître visuellement sur la coquille une ombre portée du réseau sanguin alimentant l'embryon de l'oeuf s'il est présent dans l'oeuf. Au moins une image de l'oeuf est capturée puis traitée pour déterminer, en fonction de la présence ou non de ce réseau sanguin et de sa taille, si l'oeuf est dans un état vivant ou dans un état mort.

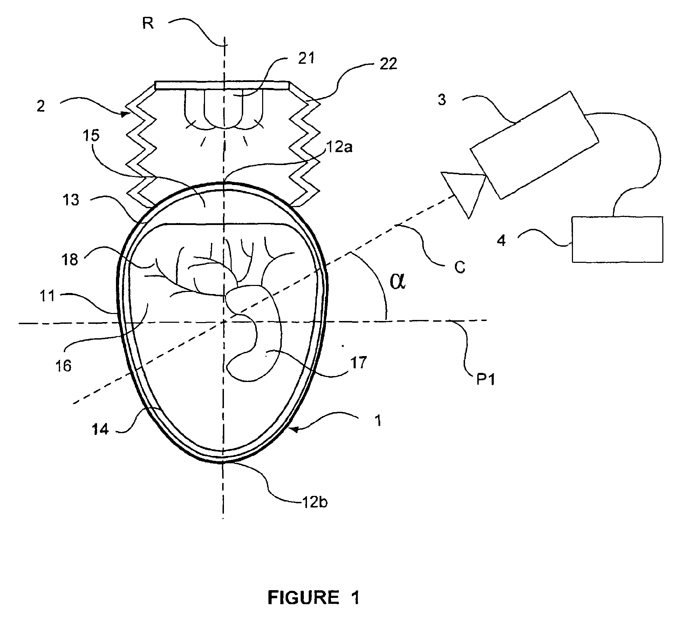

Le procédé de l'invention est illustré par la figure 1. L'oeuf miré est un oeuf fécondé 1 représenté de manière schématique. Il comprend une coquille 11 de forme ovoïde présentant une extrémité aplatie 12a et une extrémité pointue 12b. Une membrane coquillière externe très fine 13 tapisse l'intérieur de la coquille 11. Une membrane coquillière interne 14 également très fine tapisse partiellement la membrane coquillière externe 13 et s'en décolle au pôle apical de l'oeuf de manière à former une chambre à air 15 au niveau de l'extrémité aplatie 12a de l'oeuf. La chambre à air apparaît très lumineuse lorsqu'elle est éclairée par une lumière bleue. La membrane coquillière interne 14 délimite une poche 16 renfermant, outre du blanc et des liquides embryonnaires transparents en partie supérieure et du jaune d'oeuf en partie inférieure, un embryon 17 en position intermédiaire au-dessus du jaune, lequel embryon est alimenté par un réseau sanguin 18 comprenant des veines et des vaisseaux capillaires. Le réseau sanguin est disposé majoritairement contre la membrane coquillière interne 14. Il est donc très proche de la coquille et baigne dans les liquides amniotiques transparents.

L'oeuf 1 est disposé de manière à ce que son axe de révolution, noté R, soit sensiblement vertical. Un plan de référence P1, perpendiculaire à l'axe de révolution R et coupant cet axe en un point situé à mi-distance entre l'extrémité aplatie 12a et l'extrémité pointue 12b de l'oeuf, est défini. La partie de l'oeuf 1 située au-dessus du plan de référence P1, appelée partie supérieure de l'oeuf, est éclairée par une source de lumière 2 disposée au-dessus de l'oeuf sensiblement sur l'axe de révolution R. La source de lumière 2 est une source de lumière bleue dont la ou les longueurs d'onde sont comprises entre 440nm et 510nm. Cette lumière est particulièrement avantageuse pour faire apparaître les veines et vaisseaux du réseau sanguin 18 situés en périphérie de la face interne de la coquille. Cette lumière, qui est avantageusement diffractée, est absorbée par les éléments de l'oeuf transportant du sang. Lorsque l'oeuf est éclairé avec cette lumière verte ou bleue, une ombre portée du réseau sanguin se dessine sur la coquille de l'oeuf. La présence ou non de ce réseau sanguin et la dimension (épaisseur et/ou longueur) des veines du réseau permettent de déterminer l'état vivant ou mort de l'embryon. La chambre à air apparait également très lumineuse.

La source de lumière 2 est par exemple formée par une ou plusieurs diodes électroluminescentes 21. La plage des longueurs d'onde de la lumière émise par la source 11 est comprise entre 440 nm et 510 nm H510, avantageusement pour un stade précoce du cycle d'incubation, par exemple entre le 3^{ème} et le 6^{ème} jour d'incubation. La lumière émise est soit monochromatique (1 seule longueur d'ondes dans cette plage) ou soit polychromatique (plusieurs longueurs d'onde dans cette plage).

La source de lumière 2 est avantageusement équipée d'une coupelle 22 de forme tubulaire à soufflet venant en appui sur l'extrémité aplatie 12a de l'oeuf lors de son éclairement. Cette coupelle forme un guide de lumière entre la source de lumière 2 et l'oeuf 1 et permet d'éviter les fuites de lumière ainsi que des, entrées de lumière parasite. Cette coupelle est par exemple réalisée en élastomère noir.

Selon l'invention, une image de la partie supérieure de l'oeuf où apparaît l'ombre portée du réseau sanguin 18 sur la coquille est capturée par une caméra noir & blanc ou une caméra couleur 3 disposée à proximité de la partie supérieure de l'oeuf 1. L'axe de prise de vue, noté C, de la caméra 3 coupe de préférence l'axe de révolution R au niveau de son intersection avec le plan de référence P1. L'axe de prise de vue C forme un angle α, dit angle de latitude, avec le plan de référence P1. Cet angle est compris entre 0° et 70°. Si l'oeuf 1 est disposé dans un plateau comportant d'autres oeufs, les parois du plateau et les autres oeufs peuvent empêcher la capture de la partie supérieure de l'oeuf 1. Dans ce cas, on choisit un angle α dans la partie supérieure de la plage [0°,70°], par exemple un angle compris entre 30° et 70°. Une autre solution est de prévoir des moyens de levage de l'oeuf pour positionner la partie supérieure de l'oeuf dans le champ de l'objectif de la caméra.

Des moyens de traitement d'image 4 sont connectés à la caméra 3 pour traiter l'image capturée et déterminer, à partir de celle-ci, si l'embryon 17 est vivant ou mort.

Selon un premier mode de réalisation qui sera décrit en détail plus loin dans la description, le traitement effectué par les moyens de traitement d'image 4 consiste à déterminer si un réseau de lignes sombres correspondant au réseau sanguin de l'oeuf est présent dans l'image capturée, à évaluer l'épaisseur et/ou la longueur des veines et à comparer cette ou ces valeur(s) à une ou des valeurs de référence prédéterminées fonction de l'âge de l'oeuf. Si la valeur d'épaisseur évaluée et/ou la valeur de longueur évaluée est ou sont supérieure(s) à la valeur d'épaisseur de référence et/ou à la valeur de longueur de référence, l'oeuf est dans un état vivant. Sinon, il est dans un état mort.

Selon un second mode de réalisation également décrit en détail plus loin dans la description, en plus de la détermination d'un niveau de vitalité de l'embryon en fonction de la présence ou non d'un réseau sanguin dans ladite image et, en cas de présence d'un réseau sanguin, de la dimension des veines dudit réseau sanguin, l'embryon de l'oeuf est excité et une capture d'image est effectuée avant et après excitation de l'embryon. L'étape de traitement d'image du procédé consiste alors à comparer l'image avant excitation à l'image après excitation. Si l'oeuf est vivant, l'embryon réagit à l'excitation et son réseau sanguin ainsi que la masse sombre de l'embryon se déplace entre les deux images. Les deux images sont alors différentes. Si les deux images sont identiques, l'oeuf est mort.

Ces deux modes de réalisations peuvent être appliqués ensemble ou séparément. Ces deux modes de réalisation sont décrits de manière détaillée ci-après.

### Premier mode de réalisation

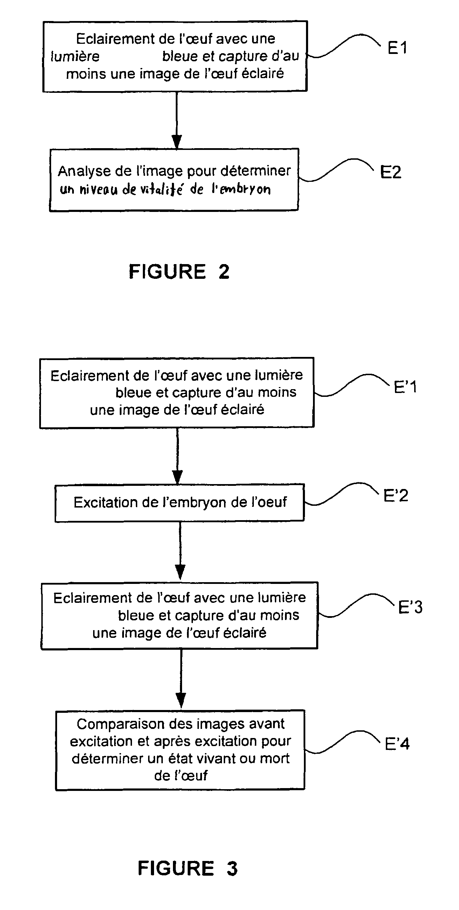

Selon ce premier mode de réalisation illustré par la figure 2, le procédé de l'invention comprend
- une étape E1 d'éclairement d'au moins une partie de l'oeuf à mirer avec une lumière bleue avec une longueur d'onde comprise entre 440 nm et 510 nm, de manière à faire apparaître visuellement sur la coquille de l'oeuf une ombre portée du réseau sanguin de l'embryon si il est présent dans l'oeuf et de capture d'au moins une image dudit oeuf éclairé; et
- une étape E2 d'analyse de l'image capturée pour déterminer un niveau de vitalité de l'embryon dudit oeuf

L'étape E2 consiste à quantifier dans l'image l'épaisseur et/ou la longueur des lignes sombres représentant l'ombre du réseau sanguin de l'oeuf sur la coquille et à comparer ces valeurs à des valeurs de référence prédéterminées qui sont fonction de l'âge de l'oeuf. Pour un âge donné, si la valeur d'épaisseur est inférieure à la valeur d'épaisseur de référence prédéterminée pour cet âge et/ou si la valeur de longueur est inférieure à la valeur de longueur de référence prédéterminée pour cet âge, l'oeuf est considéré comme mort. Sinon, il est considéré comme vivant. Ces valeurs de référence augmentent de manière spécifique avec l'âge de l'oeuf.

Selon un perfectionnement, plusieurs valeurs d'épaisseur de référence et/ou plusieurs valeurs de longueur de référence sont définies pour chaque stade du cycle d'incubation de l'oeuf afin de caractériser, de manière plus fine, le niveau de vitalité de l'embryon à un stade donné du cycle d'incubation, la règle étant que, à un âge donné et en dessous d'un seuil donné, plus l'épaisseur et/ou la longueur des veines du réseau sanguin est faible, plus le niveau de vitalité de l'embryon est faible. Au-delà de ce seuil, l'embryon est considéré comme vivant et vigoureux. Ainsi, la dimension des veines évaluée à l'étape E2 est comparée à ces différentes valeurs de référence et le résultat de cette comparaison permet de définir un niveau de vitalité de l'embryon.

Selon un perfectionnement, au moins deux images de l'oeuf éclairé sont capturées par des caméras ayant des angles de prise de vue différents. Il arrive en effet que, suivant la position de l'embryon dans l'oeuf et suivant l'angle de prise de vue de la caméra 3, le réseau sanguin ne soit pas visible ou soit peu visible dans l'image capturée par la caméra 3. On prévoit donc avantageusement de prendre une autre image de l'oeuf éclairé par une autre caméra disposée par exemple à l'opposé de la caméra 3 par rapport au plan médian vertical de l'oeuf.

Selon ce perfectionnement, l'étape E1 comprend l'éclairement de l'oeuf avec une lumière bleue et la capture d'une image I1 de l'oeuf éclairé par une première caméra, par exemple la caméra 3, et la capture d'une image I2 de l'oeuf éclairé par une deuxième caméra, non représentée sur la figure 1, lesdites première et deuxième caméras étant disposées symétriquement de part et d'autre de l'axe de révolution de l'oeuf. L'étape E2 comprend une analyse des deux images I1 et I2 pour déterminer la présence ou non d'un réseau sanguin et en déduire un état vivant ou un état mort de l'oeuf.

### Deuxième mode de réalisation

Selon ce deuxième mode de réalisation illustré par la figure 3, le procédé de l'invention comprend , en plus de la détermination d'un niveau de vitalité de l'embryon en fonction de la présence ou non d'un réseau sanguin dans la dite image et, en cas de présence d'un réseau sanguin de la dimension des veines,
- une étape E'1 d'éclairement de l'oeuf avec une lumière bleue et de capture d'au moins une image I1 de l'oeuf éclairé par une caméra,
- une étape E'2 d'excitation de l'embryon de l'oeuf par un stimulus,
- une étape E'3 d'éclairement de l'oeuf avec ladite lumière bleue et de capture, après excitation de l'embryon, d'une image I'1 de l'oeuf éclairé avec la même caméra, et
- une étape E'4 de comparaison des deux images I1 et I'1 pour en déduire l'état, vivant ou mort, de l'oeuf. Si l'embryon est vivant, le réseau sanguin et la masse opaque de l'embryon changent de position dans l'oeuf entre les images I1 et I'1. Si les images I1 et I'1 sont différentes, on peut donc en déduire que l'oeuf est dans un état vivant.

Le stimulus employé à l'étape E'2 est une perturbation temporaire du milieu environnant l'embryon. Il peut prendre la forme d'une onde lumineuse, sonore et/ou thermique ou d'une vibration provoquée par exemple par un choc ou tout autre type de stimulus provoquant un mouvement chez l'embryon. Le stimulus est par exemple un signal lumineux intense, et/ou thermique, tel qu'une lumière blanche ou un signal laser. Ce stimulus est de préférence un signal laser. Les moyens pour générer ce stimulus sont avantageusement prévus dans la source de lumière 2. Ces moyens sont par exemple une source de lumière blanche ou une diode laser disposée à coté des diodes électroluminescentes bleues de la source de lumière 2.

Selon un perfectionnement, au moins deux images de l'oeuf sont capturées sous des angles de prise de vue différents avant excitation et après excitation. Selon ce perfectionnement, l'étape E'1 comprend l'éclairement de l'oeuf avec une lumière bleue, la capture d'une image I1 de l'oeuf éclairé par une première caméra et la capture d'une image I2 de l'oeuf éclairé par une deuxième caméra. L'étape E'2 d'excitation est inchangée. L'étape E'3 comprend l'éclairement de l'oeuf avec ladite lumière bleue, la capture d'une image I'1 de l'oeuf éclairé par la première caméra et la capture d'une image I'2 de l'oeuf éclairé par la deuxième caméra. L'étape E'4 comprend alors une comparaison des deux images I1 et I'1 et/ou une comparaison des deux images I2 et I'2 pour déterminer l'état vivant ou l'état mort de l'oeuf. Si le réseau sanguin et la masse opaque de l'embryon ont changé de position entre les images I1 et I'1 ou entre les images I2 et I'2, l'oeuf est dans un état vivant.

En variante, l'étape E'4 comporte une analyse des deux images I1 et I2 et/ou des deux images I'1 et I'2 pour déterminer, à partir des lignes sombres qu'elles comportent, si l'oeuf est dans un état vivant ou mort, puis une comparaison des deux images I1 et I'1 et/ou des deux images I2 et I'2 pour valider l'état vivant ou mort déterminé par l'analyse.

Ce second mode de réalisation peut venir en complément du premier mode de réalisation pour confirmer le niveau de vitalité déterminé lors de la l'analyse de la dimension des veines du réseau sanguin.

Selon un perfectionnement applicable aux deux modes de réalisation, l'étape E2 ou E'4 comprend en outre une analyse d'au moins l'une des images I1, I2, I'1 1 et I'2 pour déterminer la position de la chambre à air 15 de l'oeuf et en déduire si l'oeuf est dans un état retourné ou un état non retourné. L'oeuf est défini comme étant dans un état retourné lorsque sa chambre à air 15 est localisée dans la partie inférieure de l'oeuf et comme étant dans un état non retourné lorsque sa chambre à air est localisée dans la partie supérieure de l'oeuf. La détection de cet état est importante pour l'injection de vaccins dans l'oeuf et/ou le décalotage de celui-ci. L'analyse de l'image consiste alors à détecter si la partie supérieure de l'oeuf comporte ou non une zone très lumineuse correspondant à la chambre à air de l'oeuf. Si une telle zone est détectée dans la partie supérieure de l'oeuf, celui-ci est dans un état non retourné. L'analyse d'image peut également permettre de voir si la chambre à air est décalée par rapport a l'axe vertical de l'oeuf.

Selon un autre perfectionnement applicable aux deux modes de réalisation, l'étape E2 ou E'4 comprend en outre une analyse d'au moins l'une des images I1, I2, I'1 et I'2 pour vérifier l'intégrité de la coquille de l'oeuf et détecter la présence éventuelle de fêlures. Ces fêlures ont pour caractéristique de laisser passer la lumière. La détection des fêlures revient alors à détecter dans les images capturées la présence de lignes lumineuses très intenses.

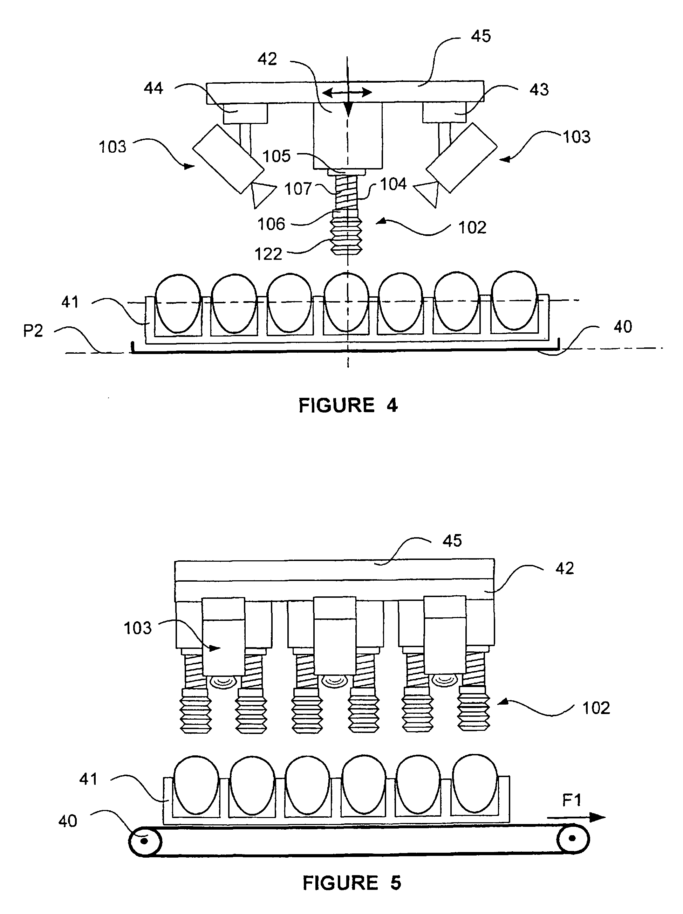

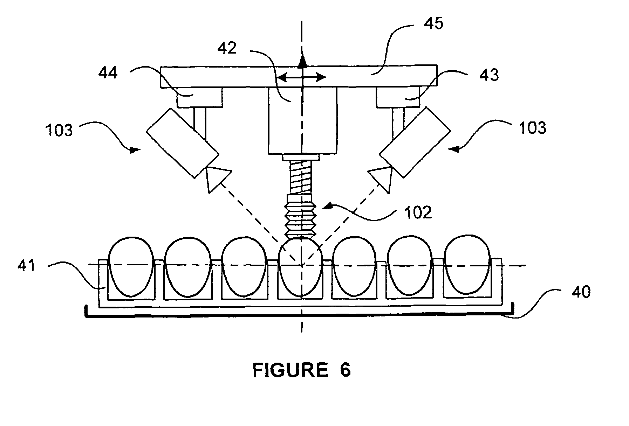

Les figures 4 à 6 représentent de manière schématique un dispositif de mirage automatique apte à mettre en oeuvre le procédé de l'invention. Les figures 4 et 5 représentent respectivement des vues de côté, respectivement transversale et parallèle à la direction d'avancement des plateaux, du dispositif en position relevée et la figure 6 représente une vue de côté analogue à celle de la figure 4 dans laquelle le dispositif est en position de mirage. Le dispositif comporte des moyens de convoyage 40 pour transporter des plateaux d'incubation d'oeufs 41, suivant un chemin de convoyage le long duquel sont disposés des moyens d'éclairement 102 et des moyens de capture d'image 103 des oeufs éclairés. Des moyens de traitement d'image, non représentés sur les figures 4 et 6, sont prévus pour traiter les images capturées par les moyens de capture d'image 103.

Les moyens de convoyage 40 comprennent par exemple un convoyeur de type à bande sans fin, définissant un plan de convoyage P2 (fig.4) de plateaux, apte à déplacer les plateaux 41 dans la direction d'avancement F1. Les plateaux comprennent par exemple des rangées longitudinales d'alvéoles, les rangées étant alignées ou décalées (alvéoles disposées en quinconce) entre elles. Dans l'exemple illustré, le plateau comprend 7 rangées alignées de 6 oeufs disposées parallèlement à direction d'avancement F1.

Les moyens d'éclairement 102 et les moyens de capture d'image 103 sont adaptés pour traiter les oeufs rangée par rangée. Les moyens d'éclairement 102 sont constitués d'une rangée de sources de lumière disposées côte à côte sur une rampe horizontale 42, dite d'éclairement. Dans l'exemple illustré, la rampe d'éclairement est placée parallèlement à la direction d'avancement F1 au dessus du chemin de convoyage. Une source de lumière 102 est prévue pour chaque oeuf d'une même rangée. Chaque source de lumière 102 comprend un tube 104 comprenant à son extrémité supérieure une embase 105 pour fixer le tube 104 à la rampe 42 et portant à son extrémité inférieure une ou plusieurs diodes électroluminescentes montées sur une douille 106. La douille 106 est montée coulissante sur le tube 104 et est sollicitée élastiquement par un ressort 107 monté entre l'embase 105 et la douille 106. La douille 106 est de préférence équipée d'une coupelle 122 en forme de soufflet pour guider la lumière produite par la ou les diodes vers l'oeuf et venir en contact avec la partie supérieure de l'oeuf lorsque la rampe d'éclairement 42 est abaissée (position de mirage).

Les moyens de capture d'image 103 sont constitués d'une pluralité de caméras disposées sur au moins une rampe horizontale 43. La rampe 43 est placée parallèlement à la direction d'avancement F1 au dessus du chemin de convoyage. Une caméra de la rampe est prévue pour chaque oeuf ou pour chaque groupe d'oeufs de la rangée disposés côte à côte. Dans l'exemple illustré, une caméra est prévue pour deux oeufs. Avantageusement, une deuxième rampe horizontale 44 munie de caméras est disposée symétriquement à la rampe 43 de caméras par rapport à la rampe de sources de lumière. Les caméras sont destinées à capturer une image de la partie supérieure des oeufs de la rangée.

Les rampes de caméras 43 et 44 et la rampe de sources de lumière 42 sont montées sur un support 45 apte à se déplacer verticalement entre une position relevée (position haute) et une position de mirage (position basse) et transversalement. Le déplacement du support 45 est commandé par des moyens de commande non représentés sur les figures 4 à 6. Ces moyens de commande commandent également l'allumage des sources de lumière et la capture d'images par les caméras. Il est à noter que ces moyens de commande peuvent être réunis avec les moyens de traitement d'image 4 dans une unité de commande centrale du dispositif.

En fonctionnement, un plateau est acheminé, sous le support 45, ledit support étant en position relevée avec les sources de lumière de la rampe d'éclairement 42 positionnées au dessus d'une rangée d'oeuf du plateau. Les moyens de commande déplacent verticalement vers le bas le support jusqu'à ce que la coupelle des sources de lumière vienne en appui sur la partie supérieure des oeufs de la rangée. Le support est alors en position de mirage (position basse). Les sources de lumière sont alors allumées. Les caméras disposées symétriquement de part et d'autre des sources de lumière capturent alors, successivement ou simultanément, des images des oeufs éclairés. Les moyens de commande déplacent ensuite verticalement vers le haut le support 45 jusqu'à sa position relevée (position haute) puis transversalement pour positionner les sources de lumière au-dessus de la rangée suivante d'oeufs. Les images capturées par les caméras sont transmises aux moyens de traitement d'image au fur et à mesure de leur capture ou après capture des images de l'ensemble des oeufs du plateau. Lorsque toutes les rangées du plateau ont été traitées, le convoyeur est actionné pour acheminer un nouveau plateau à traiter.

Dans l'exemple illustré, une seule caméra est prévue pour chaque paire d'oeufs voisins de la rangée. On prévoit alors de préférence un éclairement alternée entre les deux oeufs de la paire et une capture d'image séparée pour les deux oeufs. On éclaire d'abord un oeuf, on capture une image pour cet oeuf avec la caméra, puis on éclaire l'autre oeuf et on capture l'image pour l'autre oeuf. Ainsi, pour une rangée d'oeufs, tous les oeufs de rang pair de la rangée sont éclairés simultanément dans un premier temps puis tous les oeufs de rang impair sont éclairés simultanément dans un second temps.

Pour le traitement de plateaux décalés dans lesquels les oeufs de deux rangées successives sont disposés en quinconce, les moyens de déplacement sont en outre aptes à déplacer longitudinalement le support 45 pour passer d'une rangée à l'autre. Il est également possible de prévoir deux rampes de sources de lumière, montées sur un même support ou chacune sur un support, les sources de lumière des deux rampes étant décalées longitudinalement l'une par rapport à l'autre et chaque rampe de sources de lumière étant associée à une ou deux rampes de caméras.

Avantageusement, les sources de lumière sont munies de moyens permettant de faire varier la luminosité afin d'affiner la qualité des images prises par les caméras.

Avantageusement, plusieurs images peuvent être prises dans la même position par une même caméra avec pour seule différence entre elles soit l'intensité lumineuse des sources lumineuses, soit la durée d'exposition utilisée pour la prise d'image, afin d'affiner la qualité des détails pour l'analyse du réseau sanguin et/ou d'autres éléments accessoires contenus dans l'oeuf.

Pour la mise en oeuvre du second mode de réalisation du procédé de l'invention (avec excitation de l'embryon), les sources de lumière la rampe d'éclairement sont équipés de moyens d'excitation tels qu'une diode laser.

Selon un perfectionnement, le dispositif est équipé de moyens de levage des oeufs pour faciliter la capture des images lorsque les oeufs sont serrés, par exemple lorsqu'ils sont disposés en quinconce dans les plateaux d'incubation, ou lorsque la taille des oeufs environnant l'oeuf à analyser gène la prise d'image, par exemple lorsqu'un oeuf très gros est disposé devant l'oeuf à analyser de plus petite taille.

Bien que l'invention ait été décrite en liaison avec différents modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention comme defini dans les revendications. On peut prévoir de disposer les rampes de sources de lumière et de caméras transversalement à la direction d'avancement F1 des moyens de convoyage et de déplacer, pour passer d'une rangée d'oeufs à l'autre, les moyens de convoyage au lieu de déplacer les rampes. On peut également prévoir de traiter plusieurs rangées d'oeufs à la fois en utilisant plus de rampes de sources de lumière et rampes de caméras.

## Revendications

1. Procédé de mirage d'oeufs pour déterminer au moins un état d'au moins un oeuf fécondé contenant un embryon, **caractérisé en ce qu'**il comporte les étapes suivantes:
a) éclairement dudit oeuf avec une lumière bleue ayant une ou plusieurs longueurs d'onde comprises entre 440nm et 510 nm et capture d'au moins une image dudit oeuf éclairé; et
b) analyse de ladite image pour déterminer, en fonction de la présence ou non d'un réseau sanguin alimentant l'embryon dudit oeuf dans ladite image et, en cas de présence d'un réseau sanguin, de la dimension des veines dudit réseau sanguin, un niveau de vitalité de l'embryon dudit oeuf.

2. Procédé selon la revendication 1, **caractérisé en ce que** l'étape a) comprend l'éclairement (E1) de la partie supérieure de l'oeuf disposé de sorte que son axe de révolution soit sensiblement vertical, au moyen d'une source de lumière disposée au-dessus de l'oeuf sensiblement sur l'axe de révolution (R) dudit oeuf, et la capture (E1) d'une image de la partie supérieure de l'oeuf éclairé par une caméra (3), disposée à proximité de la source de lumière, l'axe de prise de vue (C) de la caméra formant un angle (α) compris entre 0 et 70° avec un plan de référence (P) perpendiculaire à l'axe de révolution (R) de l'oeuf et situé à mi-distance entre l'extrémité aplatie (12a) et l'extrémité pointue (12b) de l'oeuf.

3. Procédé selon l'une des revendications 1 à 2, **caractérisé en ce que** l'étape a) comprend l'éclairement (E1) dudit oeuf avec une lumière bleue, la capture (E1) d'une image I1 dudit oeuf éclairé par une première caméra et la capture d'une image I2 dudit oeuf éclairé par une deuxième caméra, lesdites première et deuxième caméras étant disposées en vis-à-vis de part et d'autre d'un plan médian de l'oeuf comprenant l'axe de révolution dudit oeuf,
et **en ce que** l'étape b) comprend une analyse (E2) des deux images I1 et I2 pour déterminer le niveau de vitalité de l'oeuf.

4. Procédé selon l'une quelconque des revendications 1 à 2 **caractérisé en ce que** l'étape a) comprend:
- l'éclairement (E'1) dudit oeuf avec une lumière bleue et la capture (E'1) d'une image I1 dudit oeuf éclairé par une caméra,
- l'excitation (E'2) de l'embryon de l'oeuf par un stimulus, et, après ladite excitation,
- l'éclairement (E'3) dudit oeuf avec ladite lumière bleue et la capture (E'3) de l'embryon, d'une image I'1 dudit oeuf éclairé par ladite caméra,
et **en ce que** l'étape b) comprend une comparaison (E'4) des deux images I1 et I'1 pour confirmer le niveau de vitalité de l'embryon.

5. Procédé selon la revendication 4, **caractérisé en ce que** l'étape a) comprend
- l'éclairement (E' 1) dudit oeuf avec une lumière bleue, la capture d'une image I1 dudit oeuf éclairé par une première caméra et la capture d'une image I2 dudit oeuf éclairé par une deuxième caméra,
- l'excitation (E'2) de l'embryon de l'oeuf par un stimulus, et
- après ladite excitation, l'éclairement (E'3) dudit oeuf avec ladite lumière bleue, la capture (E'3) d'une image I'1 dudit oeuf éclairé par la première caméra et la capture d'une image I'2 dudit oeuf éclairé par la deuxième caméra,
et **en ce que** l'étape b) comprend une comparaison (E'4) des deux images I1 et I'1 et/ou une comparaison des deux images I2 et I'2 pour confirmer le niveau de vitalité de l'embryon.

6. Procédé selon la revendication 4 ou 5, **caractérisé en ce que** le stimulus est un signal sonore et/ou lumineux et/ou thermique.

7. Procédé selon l'une quelconque des revendications précédentes, **caractérisé en ce que** l'étape b) comprend en outre une analyse de ladite ou lesdites images pour déterminer la position de la chambre à air de l'oeuf et en déduire un état retourné ou un état non retourné de l'oeuf.

8. Procédé selon l'une quelconque des revendications précédentes, **caractérisé en ce que**, pour deux oeufs voisins, l'éclairement est effectué de manière alternée entre les deux oeufs.

9. Dispositif de mirage automatique d'oeufs pour déterminer au moins un état d'un oeuf fécondé, **caractérisé en ce qu'**il comprend :
- des moyens d'éclairement (2 ;102) comprenant au moins une source de lumière bleue ayant une ou plusieurs longueurs d'onde comprises entre 440 nm et 510 nm, pour éclairer ledit oeuf;
- des moyens de capture d'image (3 ;103) pour capturer au moins une image dudit oeuf éclairé; et
- des moyens d'analyse d'image (4) pour déterminer, en fonction de la présence ou non d'un réseau sanguin alimentant l'embryon de l'oeuf dans ladite image et, en cas de présence d'un réseau sanguin, de la dimension des veines dudit réseau sanguin, un niveau de vitalité de l'embryon dudit oeuf.

10. Dispositif selon la revendication 9, **caractérisé en ce qu'**il comprend en outre :
- des moyens de convoyage (40) pour transporter, selon un plan de convoyage et une direction d'avancement, des plateaux comportant des alvéoles dans lesquelles sont disposés des oeufs à mirer,
- un support (45), disposé au-dessus des moyens de convoyage, portant les moyens d'éclairement (102) comprenant au moins une rampe de sources de lumière et les moyens de capture d'image (103) comprenant au moins une rampe de caméras, ladite rampe de sources de lumière et ladite rampe de caméras étant disposées parallèlement ou transversalement à la direction d'avancement (F1), et
- des moyens de déplacement aptes à déplacer verticalement ledit support (45) entre une position relevée et une position de mirage dans laquelle les sources de lumière viennent en appui sur la partie supérieure des oeufs positionnés en vis-à-vis dans les alvéoles.

11. Dispositif selon la revendication 10, **caractérisé en ce que** lesdits moyens de déplacement sont aptes à déplacer ledit support parallèlement et/ou transversalement à la direction d'avancement (F1).

12. Dispositif selon la revendication 10 ou 11, **caractérisé en ce que** lesdits moyens de capture d'image (103) comportent deux rampes de caméras (43) disposées symétriquement de part et d'autre de la rampe des sources de lumière, l'axe de prise de vue (C) des caméras formant un angle (α) compris entre 0 et 70° avec un plan horizontal des moyens de convoyage.

13. Dispositif selon l'une quelconque des revendications 9 à 12, **caractérisé en ce que** les moyens d'éclairement comprennent des moyens pour faire varier la luminosité de la source de lumière.

14. Dispositif selon l'une quelconque des revendications 9 à 13, **caractérisé en ce que** les moyens d'éclairement comprennent des moyens (122) pour guider la lumière produite par la source de lumière vers l'oeuf.

## Claims

1. A method for candling eggs in order to determine at least a state of at least a fertilized egg containing an embryo, **characterized in that** it comprises the following steps:
a) illumination of said egg with blue light having one or several wavelengths ranging between 440 nm and 510 nm and acquisition of at least one image of said illuminated egg; and
b) analysis of said image in order to determine a vitality level of the embryo of said egg based on the presence or not of a blood network supplying the embryo of said egg in said image and, in the case of the presence of a blood network, on the size of the veins of said blood network.

2. The method according to claim 1, **characterized in that** step a) comprises the illumination (E1) of the upper portion of the egg arranged such that its revolution axis is substantially vertical, by means of a light source arranged above the egg substantially on the revolution axis (R) of said egg, and the acquisition (E1) of an image of the upper portion of the illuminated egg by a camera (3), arranged in the vicinity of the light source, the image acquisition axis (C) of the camera forming an angle (α) ranging between 0 and 70° with a reference plane (P) perpendicular to the revolution axis (R) of the egg and located at mid-distance from the flattened end (12a) and the narrow end (12b) of the egg.

3. The method according to any one of claims 1 to 2, **characterized in that** step a) comprises the illumination (E1) of said egg with a blue light, the acquisition (E1) of an image I1 of said illuminated egg by a first camera and the acquisition of an image I2 of said illuminated egg by a second camera, said first and second cameras being arranged facing each other on either side of a median plane of the egg comprising the revolution axis of said egg,
and **in that** the step b) comprises an analysis (E2) of both images I1 and I2 to determine the vitality level of the egg.

4. The method according to any one of claims 1 to 2, **characterized in that** step a) comprises:
- the illumination (E'1) of said egg with a blue light and the acquisition (E'1) of an image I1 of said illuminated egg by a camera,
- the excitation (E'2) of the embryo of the egg by a stimulus, and, after said excitation,
- the illumination (E'3) of said egg with said blue light and the acquisition (E'3) of the embryo, of an image I'1 of said illuminated egg by said camera,
and **in that** step b) comprises a comparison (E'4) of both images I1 and I'1 to confirm the vitality level of the embryo.

5. The method according to claim 4, **characterized in that** step a) comprises
- the illumination (E'1) of said egg with a blue light, the acquisition of an image I1 of said illuminated egg by a first camera and the acquisition of an image I2 of said illuminated egg by a second camera,
- the excitation (E'2) of the embryo of the egg by a stimulus, and
- after said excitation, the illumination (E'3) of said egg with said blue light, the acquisition (E'3) of an image I'1 of said illuminated egg by the first camera and the acquisition of an image I'2 of said illuminated egg by the second camera,
and **in that** step b) comprises a comparison (E'4) of both images I1 and I'1 and/or a comparison of both images I2 and I'2 to confirm the vitality level of the embryo.

6. The method according to claim 4 or 5, **characterized in that** the stimulus is a sound and/or light and/or thermal signal.

7. The method according to any one of the preceding claims, **characterized in that** step b) further comprises an analysis of said image(s) in order to determine the position of the air cell of the egg and to deduce an inverted state or an uninverted state of the egg.

8. The method according to any one of the preceding claims, **characterized in that**, for two neighbouring eggs, the illumination is carried out in an alternated manner between both eggs.

9. An automatic egg candling device for determining at least a state of a fertilized egg, **characterized in that** it comprises:
- illumination means (2 ;102) comprising at least a blue light source having one or several wavelengths between 440 and 510 nm for illuminating the egg ;
- image acquisition means (3 ;103) for acquiring at least one image of said illuminated egg ; and
- image analysis means (4) for determining, based on the presence or not of a blood network supplying the embryo of the egg in said image and, in the case of presence of a blood network, of the size of the veins of said blood network, a vitality level of the embryo of said egg.

10. The device according to claim 9, **characterized in that** it further comprises:
- conveying means (40) for transporting, according to a conveyor plan and a direction of movement, trays comprising cells wherein are arranged eggs to be candled,
- a support (45) arranged above the conveying means, bearing the illumination means (102) comprising at least a ramp of light sources and the image acquisition means (103) comprising at least a ramp of cameras, said ramp of light sources and said ramp of cameras being arranged parallely or transversally to the direction of movement (F1) and
- displacement means able to vertically move the support (45) between a raised position and a candling position wherein the light sources abut against the upper portion of the eggs positioned facing each other in the cells.

11. The device according to claim 10, **characterized in that** said traveling means are able to move said support parallely and/or transversally to the direction of movement (F1).

12. The device according to claim 10 or 11, **characterized in that** said image acquisition means (103) comprise two ramps of cameras (43) arranged symmetrically on either side of the ramp of light sources, the acquisition axis (C) of the cameras forming an angle (α) ranging between 0 and 70° with a horizontal plane of the conveying means.

13. The device according to any one of claims 9 to 12, **characterized in that** the illumination means comprise means to make the luminosity of the light source vary.

14. The device according to any one of claims 9 to 13, **characterized in that** the illumination means comprises means (122) for guiding the light produced by the light source towards the egg.

## Patentansprüche

1. Verfahren zum Durchleuchten von Eiern, um mindestens einen Zustand mindestens eines befruchteten, ein Embryo enthaltenden Eis festzustellen, dadurch gekennzeichn e t , dass es die folgenden Schritte umfasst:
a) Beleuchten des Eis mit einem blauen Licht einer oder mehrerer Wellenlängen zwischen 440 und 510 nm und Erfassen mindestens einer Abbildung des beleuchteten Eis; und
b) Analyse der Abbildung zum Bestimmen eines Vitalitätsniveaus des Embryos des Eis in Abhängigkeit vom Vorhandensein oder Nichtvorhandensein eines das Embryo des Eis versorgenden Blutgefäßnetzes in der Abbildung und, im Falle des Vorhandenseins eines Blutgefäßnetzes, von der Größe der Venen des Blutgefäßnetzes.

2. Verfahren nach Anspruch 1, **dadurch gekennzeichnet, dass** der Schritt a) das Beleuchten (E1) des oberen Teils des Eis mittels einer Lichtquelle umfasst, wobei das Ei so angeordnet ist, dass dessen Rotationsachse im Wesentlichen vertikal verläuft, und wobei die Lichtquelle oberhalb des Eis sowie im wesentlichen auf dessen Rotationsachse (R) angeordnet ist, und das Erfassen (E1) einer Abbildung des oberen, beleuchteten Teils des Eis durch eine in der Nähe der Lichtquelle angeordnete Kamera (3) umfasst, wobei die optische Achse (C) der Kamera einen Winkel (α) zwischen 0 und 70° mit einer zur Rotationsachse (R) des Eis senkrecht stehenden Bezugsebene (P) einschließt und auf halber Strecke zwischen dem abgeflachten Ende (12a) und dem spitzen Ende (12b) des Eis liegt.

3. Verfahren nach einem der Ansprüche 1 bis 2, **dadurch gekennzeichnet, dass** der Schritt a) das Beleuchten (E1) des oberen Teils des Eis mit einem blauem Licht, das Erfassen (E1) einer Abbildung I1 des beleuchteten Eis durch eine erste Kamera und das Erfassen einer Abbildung I2 des beleuchteten Eis durch eine zweite Kamera umfasst, wobei die erste und zweite Kamera sich gegenüber auf beiden Seiten einer medialen, die Rotationsachse des Eis einschließenden Ebene des Eis angeordnet sind,
und dadurch, dass der Schritt b) eine Analyse (E2) der beiden Abbildungen I1 und I2 umfasst, um das Vitalitätsniveau des Eis zu bestimmen.

4. Verfahren nach einem der Ansprüche 1 bis 2, **dadurch gekennzeichnet, dass** der Schritt a) umfasst:
- das Beleuchten (E'1) des Eis mit einem Blauen Licht und das Erfassen (E'1) einer Abbildung I1 des beleuchteten Eis durch eine Kamera,
- die Anregung (E'2) des Embryos durch einen Stimulus und, nach der Anregung,
- das Beleuchten (E'3) des Eis mit dem blauen Licht und das Erfassen (E'3) des Embryos aus einer Abbildung I'1 des durch die Kamera beleuchteten Eis,
und dadurch, dass der Schritt b) einen Vergleich (E'4) der beiden Abbildungen I1 und I'1 umfasst, um das Vitalitätsniveau des Embryos zu bestätigen.

5. Verfahren nach Anspruch 4, **dadurch gekennzeichnet, dass** der Schritt a) umfasst:
- das Beleuchten (E'1) des Eis mit einem Blauen Licht, das Erfassen einer Abbildung I1 des beleuchteten Eis durch eine erste Kamera und das Erfassen einer Abbildung I2 des beleuchteten Eis durch eine zweite Kamera
- die Anregung (E'2) des Embryos durch einen Stimulus und,
- nach der Anregung, das Beleuchten (E'3) des Eis mit dem blauen Licht, das Erfassen (E'3) einer Abbildung I'1 des durch die erste Kamera beleuchteten Eis und das Erfassen einer Abbildung I'2 des durch die zweite Kamera beleuchteten Eis,
und dass der Schritt b) einen Vergleich (E'4) der beiden Abbildungen I1 und I'1 und/oder einen Vergleich der beiden Abbildungen I2 und I'2 umfasst, um das Vitalitätsniveau des Embryos zu bestätigen.

6. Verfahren nach Anspruch 4 oder 5, **dadurch gekennzeichnet, dass** der Stimulus ein akustisches und/oder optisches und/oder thermisches Signal ist.

7. Verfahren nach einem der vorstehenden Ansprüche, **dadurch gekennzeichnet, dass** der Schritt b) unter anderem eine Analyse der Abbildung(en) umfasst, um die Position der Luftkammer des Eis zu bestimmen und daraus auf einen umgedrehten oder nicht umgedrehten Zustand des Eis zu schließen.

8. Verfahren nach einem der vorstehenden Ansprüche, **dadurch gekennzeichnet, dass** das Beleuchten für zwei benachbarte Eier zwischen den beiden Eiern abwechselnd durchgeführt wird.

9. Vorrichtung zum automatischen Durchleuchten von Eiern, um mindestens einen Zustand eines befruchteten Eis festzustellen, **dadurch gekennzeichnet, dass** es umfasst:
- Beleuchtungsmittel (2; 102) umfassend mindestens eine blaue Lichtquelle mit einer oder mehreren Wellenlängen im Bereich zwischen 440 und 510 nm zum Beleuchten des Eis;
- Bilderfassungsmittel (3; 103) zum Erfassen mindestens einer Abbildung des beleuchteten Eis; und
- Bildanalysemittel (4), um ein Vitalitätsniveau des Embryos des Eis zu bestimmen in Abhängigkeit vom Vorhandensein oder Nicht-Vorhandensein eines das Embryo des Eis versorgenden Blutgefäßnetzes in der Abbildung und, im Falle des Vorhandenseins eines Blutgefäßnetzes, von der Größe der Venen des Blutgefäßnetzes.

10. Vorrichtung nach Anspruch 9, **dadurch gekennzeichnet, dass** sie unter Anderem umfasst:
- Fördermittel (40), um Tabletts mit Kammern, in denen die zu beleuchtenden Eier angeordnet sind, gemäß eines Förderplans und einer Förderrichtung zu transportieren,
- einen oberhalb der Fördermittel angeordneten Träger (45), der die Beleuchtungsmittel (102), welche mindestens eine Lichtquellenleiste umfassen, sowie die Bilderfassungsmittel (103), welche mindestens eine Kameraleiste umfassen, trägt, wobei die Lichtquellenleiste und die Kameraleiste zur Förderrichtung (F1) parallel oder quer angeordnet sind, und
- Verlagerungsmittel zur vertikalen Verlagerung des Trägers (45) zwischen einer angehobenen Stellung und einer Durchleuchtungsstellung, in der sich die Lichtquellen auf dem oberen Teil der ihnen gegenüber in den Kammern angeordneten Eier abstützen.

11. Vorrichtung nach Anspruch 10, **dadurch gekennzeichnet, dass** die Verlagerungsmittel dazu geeignet sind, den Träger parallel oder quer zur Förderrichtung (F1) zu verlagern.

12. Vorrichtung nach Anspruch 10 oder 11, **dadurch gekennzeichnet, dass** die Bilderfassungsmittel (103) zwei Kameraleisten (43) umfassen, die symmetrisch auf beiden Seiten der Lichtquellenleiste angeordnet sind, wobei die optische Achse (C) der Kameras einen Winkel (α) zwischen 0 und 70° mit einer horizontalen Ebene der Fördermittel einschließt.

13. Vorrichtung nach einem der Ansprüche 9 bis 12, **dadurch gekennzeichnet, dass** die Beleuchtungsmittel Einrichtungen zur Veränderung der Helligkeit der Lichtquellen umfassen.

14. Vorrichtung nach einem der Ansprüche 9 bis 13, **dadurch gekennzeichnet, dass** die Beleuchtungsmittel Einrichtungen (122) umfassen, um das durch die Lichtquelle erzeugte Licht zum Ei zu führen.
